# DEMANDE DE BREVET EUROPEEN

(11) **EP 1 169 997 A1**
(43) Date de publication de la demande: **09.01.2002**
(21) Numéro de dépôt: 01401503.6
(22) Date de dépôt: 11.06.2001
(51) Int. Cl.: A61K 7/027, A61K 7/46

(54) **Composition cosmétique solide parfumée transparente**

(30) Priorité: 07.07.2000 FR 0008913
(71) Demandeur: L'OREAL, 75008 Paris (FR)
(72) Inventeur: Bara, Isabelle, 75013 Paris (FR)
(74) Mandataire: Rasson, Catherine

(57) **Abrégé**

L'invention se rapporte à une composition cosmétique solide transparente contenant dans un milieu physiologiquement acceptable, au moins une substance odorante (parfum ou arôme) en une quantité efficace pour parfumer les matières kératiniques, et une phase grasse liquide contenant au moins un polymère de masse moléculaire moyenne en poids allant de 1.000 à 30.000, comportant a) un squelette polymérique ayant des motifs de répétition hydrocarbonés pourvus d'au moins un hétéroatome, et b) au moins une chaîne grasse pendante et/ou terminale éventuellement fonctionnalisées, ayant de 12 à 120 atomes de carbone et étant liée à ces motifs.

Le polymère utilisé dans la composition de l'invention permet l'obtention d'un solide transparent ou translucide plus ou moins malléable (stick ou produit coulé en coupelle ou en pot), ayant une dureté dont la force va de 5 à 600 g, dont l'application sur la peau conduit à avoir une libération du parfum, contrôlée dans le temps.

## Description

La présente invention se rapporte à une composition cosmétique solide transparente, éventuellement colorée, véhiculant des substances odorantes (parfums ou arômes), destinée à parfumer la peau, y compris le cuir chevelu, et/ou les lèvres des êtres humains, contenant une phase grasse liquide gélifiée par un polymère particulier, ladite composition se présentant sous forme de produits coulés ou de sticks, et à l'utilisation notamment cosmétique de ladite composition, dont l'application conduit à une rémanence du parfum.

On sait qu'un parfum est l'association de différentes substances odorantes qui s'évaporent à des périodes différentes. Chaque parfum présente ce que l'on appelle une « note de tête » qui est l'odeur diffusant en premier lors de l'application du parfum ou lors de l'ouverture du récipient le contenant, une « note de coeur ou corps » qui correspond au parfum complet (émission pendant quelques heures après la « note de tête ») et une « note de fond » qui est l'odeur la plus persistante (émission pendant plusieurs heures après la « note de coeur »). La persistance de la note de fond correspond à la rémanence du parfum.

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

Il est courant d'incorporer du parfum dans un certain nombre de produits ou compositions, en particulier cosmétiques et dermatologiques. Or, selon la nature de ces produits ou compositions, il n'est pas toujours aisé d'incorporer un parfum et/ou de conserver les effets olfactifs recherchés.

Par ailleurs, il existe des produits parfumants destinés à parfumer notamment la peau et/ou les cheveux. La plupart des produits parfumants sont présentés sous forme de liquide dont le parfum est solubilisé par de l'alcool (éthanol). En outre, il existe sur le marché des parfums solides sans alcool présentés sous forme de coupelles, qui permettent d'offrir au consommateur un nouveau geste pour se parfumer, et qui présentent aussi l'avantage d'être moins irritants et non inflammables par rapport au support alcoolique. Un autre avantage d'une forme solide est de pouvoir appliquer du parfum à un endroit précis, ce que ne permet pas toujours un spray, et d'éviter les éclaboussures éventuelles du parfum.

Les « solides » parfumants constituant l'état de la technique sont des formes anhydres, riches en « corps gras » (ou « baume parfumant ») qui facilitent une bonne solubilité des parfums, ou bien des formes riches en poudre, telles que des poudres compressées proches des poudres compactes utilisées dans le maquillage. Ces formes solides sont opaques, soit du fait qu'elles sont réalisées avec des cires dont le réseau cristallin apporte une certaine opacité, soit du fait qu'elles contiennent des poudres non transparentes.

La transparence ou translucidité est un effet particulièrement recherché et apprécié qui correspond à une tendance actuelle commune à d'autres secteurs, tels que les textiles ou le design. Le phénomène de la transparence peut aussi permettre l'incorporation d'objets à l'intérieur du solide, visibles à l'oeil nu, tels que des éléments particulaires (paillettes, granulés, sphères non miscibles, etc). De plus, ces produits transparents peuvent être superposés à des produits de maquillage « classiques » colorés sans en modifier la couleur d'origine, tels que les rouges à lèvres, pour simplement véhiculer une bonne odeur sur les lèvres.

Aussi, il subsiste le besoin de produits parfumants, en particulier cosmétiques, ne présentant pas les inconvénients des produits de l'art antérieur, et notamment le besoin de produits parfumants complètement transparents, éventuellement colorés, pour apporter au consommateur un nouveau visuel d'une composition parfumée, permettant en outre d'augmenter la rémanence du parfum sur les matières kératiniques sans que ce parfum se dégrade, en particulier au contact des autres constituants de la composition. Les matières kératiniques sur lesquelles on peut appliquer une telle composition sont notamment la peau, les lèvres et les phanères, la composition s'appliquant plus spécialement sur la peau.

La difficulté à résoudre pour répondre à ce besoin est de réaliser une composition solide (ne s'écoulant pas sous son propre poids) tout en incorporant une forte concentration de parfum ou d'arôme, et ce sans affecter la transparence de la composition solide, l'incorporation d'un parfum en une quantité assez importante aboutissant généralement à un produit opaque.

De façon étonnante, la demanderesse a trouvé que des polymères particuliers permettaient de gélifier des huiles et d'obtenir des solides transparents tout en permettant l'incorporation de fort taux de parfums ou d'arômes sans nuire à la transparence du système. Comme polymères, on peut utiliser soit un polymère comportant un squelette polymérique ayant des motifs répétitifs avec des hétéroatomes et au moins une chaîne grasse pendante et/ou terminale soit un polyamide comportant un squelette polymérique ayant des motifs répétitifs amide et éventuellement au moins une chaîne grasse pendante et/ou terminale.

Ainsi, l'invention a pour objet une composition cosmétique solide anhydre transparente contenant dans un milieu physiologiquement acceptable, au moins 2 % en poids par rapport au poids total de la composition, d'une ou plusieurs substances odorantes (parfum ou arôme), et une phase grasse liquide contenant au moins un polymère de masse moléculaire moyenne en poids allant de 1.000 à 30.000, comportant a) un squelette polymérique ayant des motifs de répétition hydrocarbonés pourvus d'au moins un hétéroatome, et b) au moins une chaîne grasse pendante et/ou terminale éventuellement fonctionnalisée, ayant de 12 à 120 atomes de carbone et étant liée à ces motifs.

L'invention a aussi pour objet une composition cosmétique solide anhydre transparente contenant dans un milieu physiologiquement acceptable, au moins 2 % en poids d'au moins une substance odorante, et une phase grasse liquide contenant au moins un polyamide de masse moléculaire moyenne en poids allant de 1.000 à 30.000, comportant a) un squelette polymérique ayant des motifs répétitifs amide, et b) éventuellement au moins une chaîne grasse pendante et/ou terminale éventuellement fonctionnalisée, ayant de 12 à 120 atomes de carbone et étant liée à ces motifs amide.

La quantité de substance(s) odorante(s) dans la composition de l'invention contenant un polyamide va de préférence de 2 à 15 %, mieux de 3 à 12 % et encore mieux de 4 à 10 % en poids par rapport au poids total de la composition.

On entend par milieu physiologiquement acceptable dans la composition de l'invention, un milieu non toxique et susceptible d'être appliqué sur la peau (y compris l'intérieur des paupières), les lèvres, les ongles ou les cheveux d'êtres humains.

Par ailleurs, on entend par « composition solide » au sens de la présente invention, toute composition ne s'écoulant pas sous son propre poids, malléable ou non, présentant une dureté dont la force va de 5 à 600 g (gramme), mieux de 10 à 450 g et encore mieux de 50 à 450 g. Cette dureté peut être mesurée selon une méthode de pénétration d'une sonde dans ladite composition et en particulier à l'aide d'un analyseur de texture (par exemple TA-XT2 de la société Rhéo) équipé d'un cylindre en inox de 2,5 cm de haut et 2 mm de diamètre. La mesure de dureté est effectuée à environ 20°C au centre de 5 échantillons de la dite composition. Le cylindre est introduit dans chaque échantillon à une pré-vitesse de 2 mm/s puis à une vitesse de 1 mm/s et enfin à une post-vitesse de 2 mm/s, le déplacement total étant de 2 mm, la surface de l'échantillon étant au moins 10 fois supérieure au diamètre de la sonde et son épaisseur étant d'au moins 1 cm. La valeur relevée de la dureté est celle du pic maximum.

La composition de l'invention est apte à s'appliquer directement sur un support, c'est-à-dire qu'elle n'a pas besoin d'être mouillée pour s'appliquer sur le support et notamment sur la peau. On entend par "support" de la composition selon l'invention, toute surface sur laquelle on peut faire une application topique, notamment la peau, les fibres kératiniques telles que les cils et les cheveux, le cuir chevelu et les muqueuses telles que les lèvres.

La composition selon l'invention est transparente. On entend ici par « composition transparente », une composition transparente à translucide, c'est-à-dire telle qu'elle ait une valeur de turbidité inférieure à 800 NTU, et de préférence inférieure à 600 NTU. Les NTU (Nephelometric Turbidity Units) sont les unités de mesure de la turbidité d'une composition. La mesure de turbidité peut être faite par exemple avec un turbidimètre model 2100P de la société HACH COMPAGNY, les tubes utilisés pour la mesure étant référencés AR397A cat 24347-06. Les mesures sont effectuées à température ambiante (20°C à 25°C). La valeur de la turbidité dépend de la transparence du produit et aussi de sa couleur s'il contient un colorant. Ainsi, une composition transparente bleue peut avoir une valeur de turbidité allant jusqu'à 750 NTU alors qu'une composition transparente rouge peut avoir une valeur de turbidité allant jusqu'à 200 NTU.

Le polymère utilisé dans la composition selon l'invention permet d'obtenir une composition solide, malléable ou non, ayant une résistance et une rigidité particulièrement satisfaisantes tout en laissant un bon dépôt sur la peau. La composition de l'invention se présente en particulier sous forme sous forme de stick transparent ou de produit coulé transparent en coupelle ou en pot.

On entend par « substance odorante » dans la présente demande, tout parfum ou tout arôme susceptible de dégager une odeur.

Par phase grasse liquide, au sens de la présente demande, on entend une phase grasse liquide à température ambiante (environ 25°C) et à la pression atmosphérique (environ 760 mm de Hg), composée d'un ou plusieurs corps gras liquides à température ambiante, appelés aussi huiles, compatibles entre eux.

La phase grasse liquide est structurée par le polymère, et cette structuration (ou gélification) de la phase grasse liquide est modulable selon la nature du polymère utilisé. Cette structuration (ou gélification) de la phase grasse liquide permet en particulier une libération de la substance odorante et notamment du parfum, cette libération se produisant lors de l'application sur le support (la peau notamment) et aussi de manière prolongée dans le temps, c'est-à-dire que le polymère utilisé dans la composition de l'invention favorise une bonne rémanence de la substance odorante dont l'odeur persiste plus longtemps.

Ainsi, l'invention a aussi pour objet l'utilisation d'une quantité suffisante d'au moins un polymère de masse moléculaire moyenne en poids allant de 1.000 à 30.000, comportant a) un squelette polymérique ayant des motifs de répétition hydrocarbonés pourvus d'au moins un hétéroatome, et b) au moins une chaîne grasse pendante et/ou terminale éventuellement fonctionnalisée, ayant de 12 à 120 atomes de carbone et étant liée à ces motifs hydrocarbonés, dans une composition contenant une substance odorante dans un milieu physiologiquement acceptable, pour augmenter la rémanence de ladite substance odorante. La dite composition est notamment une composition cosmétique.

L'invention a encore pour objet l'utilisation d'une quantité suffisante d'au moins un polyamide de masse moléculaire moyenne en poids allant de 1.000 à 30.000, comportant a) un squelette polymérique ayant des motifs répétitifs amide, et b) éventuellement au moins une chaîne grasse pendante et/ou terminale éventuellement fonctionnalisée, ayant de 12 à 120 atomes de carbone et étant liée à ces motifs amide, dans une composition contenant une substance odorante dans un milieu physiologiquement acceptable, pour augmenter la rémanence de ladite substance odorante. La dite composition est notamment une composition cosmétique.

Par « quantité efficace », on entend en pratique une quantité d'au moins 2 % en poids, de préférence d'au moins 3 % en poids et mieux d'au moins 4 % en poids par rapport au poids total de la composition. La quantité de substance(s) odorante(s) peut aller par exemple de 2 à 15 % en poids, mieux de 3 à 12 % en poids et encore mieux de 4 à 10 % en poids par rapport au poids total de la composition.

Le polymère structurant de la composition de l'invention est un solide non déformable à température ambiante (25°C). Il est capable de structurer la composition sans l'opacifier.

Par « polymère », on entend au sens de l'invention un composé ayant au moins 2 motifs de répétition.

Par "chaînes fonctionnalisées" au sens de l'invention, on entend une chaîne alkyle comportant un ou plusieurs groupes fonctionnels ou réactifs notamment choisis parmi les groupes hydroxyle, éther, oxyalkylène ou polyoxyalkylène, carboxyle, amine, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor. Les chaînes grasses comprennent de 12 à 120 atomes de carbone et de préférence de 12 à 68 atomes de carbone.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun de un à plusieurs hétéroatomes non pendants et se trouvant dans le squelette polymérique. Ces hétéroatomes sont choisis parmi les atomes d'azote, de soufre, de phosphore et leurs associations, associés éventuellement à un ou plusieurs atomes d'oxygène. Les motifs peuvent en outre comprendre un groupement polaire du type carbonyle.

Ces motifs à hétéroatome sont en particulier des motifs amide formant un squelette du type polyamide, des motifs carbamate et/ou urée formant un squelette polyuréthane, polyurée et/ou polyurée-uréthane. De préférence, ces motifs sont des motifs amide.

Avantageusement, la ou les chaînes pendantes sont liées directement à l'un au moins des hétéroatomes du squelette polymérique.

Entre les motifs hydrocarbonés, le polymère peut comprendre des motifs siliconés ou des motifs oxyalkylénés.

En outre, le polymère de la composition de l'invention comprend avantageusement de 40 à 98 % et mieux de 50 à 95 % de chaînes grasses par rapport au nombre total des motifs à hétéroatome et des chaînes grasses. La nature et la proportion des motifs à hétéroatome est fonction de la nature de la phase grasse liquide et est en particulier similaire à la nature de la phase grasse. Ainsi, plus les motifs à hétéroatome sont polaires et en proportion élevée dans le polymère, ce qui correspond à la présence de plusieurs hétéroatomes, plus le polymère a de l'affinité avec les huiles polaires. En revanche, plus les motifs à hétéroatome sont peu polaires voire apolaires ou en proportion faible, plus le polymère a de l'affinité avec les huiles apolaires.

Avantageusement, la composition de l'invention ne contient pas de résine de silicone à motifs siloxysilicate ou de silice triméthylée, afin de préserver les propriétés de confort de la composition.

Selon un mode de réalisation de l'invention, le polymère structurant est un polyamide comportant éventuellement au moins une chaîne grasse pendante et/ou terminale éventuellement fonctionnalisée. Quand elles sont présentes, de préférence, les chaînes grasses pendantes sont liées à l'un au moins des atomes d'azote des motifs amide. En particulier, les chaînes grasses de ce polyamide représentent de 40 à 98 % du nombre total des motifs amide et des chaînes grasses, et mieux de 50 à 95 %.

Comme polymères structurant préférés utilisables dans la composition de l'invention, on peut citer les polyamides ramifiés par des chaînes grasses pendantes et/ou des chaînes grasses terminales ayant de 12 à 120 atomes de carbone et notamment de 12 à 68 atomes de carbone, les chaînes grasses terminales étant liées au squelette polyamide par des groupes ester. Ces polymères sont plus spécialement ceux décrits dans le document US-A-5783657 de la société Union Camp. Chacun de ces polymères satisfait notamment à la formule (I) suivante : 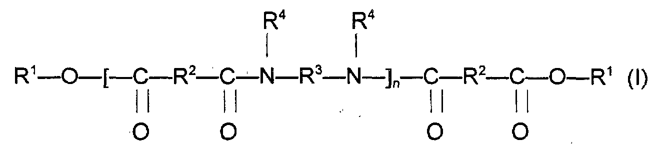 dans laquelle n désigne un nombre entier de motifs amide, tel que le nombre de groupes ester représente de 10 à 50 % du nombre total des groupes ester et amide ; R¹ est à chaque occurrence indépendamment un groupe alkyle ou alkényle ayant au moins 4 atomes de carbone ; R² représente à chaque occurrence indépendamment un groupe hydrocarboné en C₄ à C₄₂ à condition que au moins 50 % des groupes R² représentent un groupe hydrocarboné en C₃₀ à C₄₂ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5. De préférence, R¹ est un groupe alkyle en C₁₂ à C₂₂ et de préférence en C₁₆ à C₂₂. Avantageusement, R² peut être un groupe hydrocarboné alkylène notamment) en C₁₀ à C₄₂ ayant une structure d'acide gras polymérisé ou de dimère dont les groupements acide carboxylique ont été enlevés (ces groupements servant à la formation de l'amide). De préférence, 50 % au moins et mieux 75 % au moins des R² sont des groupes ayant de 30 à 42 atomes de carbone. Les autres R² sont des groupes hydrogénés en C₄ à C₁₉ et même en C₄ à C₁₂. De préférence, R³ représente un groupe hydrocarboné en C₂ à C₃₆ ou un groupe polyoxyalkyléné et R⁴ représente un atome d'hydrogène. De préférence, R³ représente un groupe hydrocarboné en C₂ à C₁₂. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

De façon avantageuse, le polymère de la composition de l'invention comprend une masse moléculaire moyenne en poids allant de 2.000 à 20.000 et mieux de 2.000 à 10.000.

Selon l'invention, la structuration de la phase grasse liquide peut être obtenue à l'aide d'un ou plusieurs polymères, et en particulier à l'aide d'un ou plusieurs polymères de formule (I). En général, les polymères de formule (I) se présentent sous forme de mélanges de polymères, ces mélanges pouvant en outre contenir un produit de synthèse correspondant à un composé de formule (I) où n vaut 0, c'est-à-dire un diester.

A titre d'exemple de polymères utilisables dans la composition selon l'invention, on peut citer les produits commerciaux fabriqués et/ou vendus par la société Bush Boake Allen sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont des copolymères d'un diacide en C₃₆ condensé sur l'éthylène diamine, estérifiés par l'alcool cétylstéarylique ou l'alcool stéarylique, de masse moléculaire moyenne d'environ 4.000 à 6.000.

Comme polymère structurant utilisable dans l'invention, on peut encore citer les résines polyamides résultant de la condensation d'un acide dicarboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles fabriqués et/ou commercialisées sous la marque Versamid® par les sociétés General Mills, Inc. et Cognis (Versamid 930, 744 ou 1655) ou sous la marque Onamid®, notamment Onamid S ou C, par la société Olin Mathieson Chemical Corp.. Ces résines ont une masse moléculaire moyenne en poids allant de 6.000 à 9.000. Pour plus d'information sur ces polyamides, on peut se référer aux documents US-A-3,645,705 et US-A-3,148,125. Plus spécialement, on utilise les Versamid® 930 ou 744.

On peut aussi utiliser les produits fabriqués et/ou commercialisés sous les dénomination Casamid® (Casamid 872, 876, 879) par la société Swan : ces polymères sont des polyamides de dimères d'acides gras et de diamines aliphatiques.

On peut aussi utiliser les polyamides à base d'acides gras dimères fabriqués et/ou vendus par la société Arizona sous les références Uni-Rez (110, 120, 118, 126, 138, 141) et le produit fabriqué et/ou vendu sous la référence Macromelt 6212 par la société Henkel. Pour plus d'information sur ces polyamides, on peut se référer au document US-A-5,500,209.

On peut bien sûr utiliser des mélanges de polymères indiqués ci-dessus.

Les polymères structurant de la composition de l'invention ont avantageusement une température de ramollissement supérieur à 70°C et pouvant aller jusqu'à 190°C. De préférence, ils présentent une température de ramollissement allant de 80 à 130°C. Ces polymères sont en particulier des polymères non cireux.

La composition contient une quantité de polymère(s), en matière active, allant de préférence de 0,5 à 50 % en poids, mieux de 5 à 40 % en poids et encore mieux de 10 à 30 % en poids par rapport au poids total de la composition.

Comme substance odorante, on peut utiliser dans la composition de l'invention, les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance odorante d'origine synthétique, on peut citer par exemple les composés du type ester, éther, aldéhyde, cétone, alcool aromatique et hydrocarbure.

Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.

Comme éthers, on peut citer le benzyléthyléther.

Comme aldéhydes, on peut citer par exemple les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal.

Comme cétones, on peut citer par exemple les ionones comme l'alpha-isométhylionone, et la méthylcédrylcétone.

Parmi les alcool aromatiques et notamment terpéniques, on peut citer l'anéthol, le citronellol, l'eugénol, l'isoeugénol, le géraniol, le linalol, le phényléthylalcool et le terpinéol.

Comme hydrocarbures, on peut citer notamment les terpènes. Ces composés se présentent souvent sous forme de mélange de deux ou plus de ces substances odorantes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

On utilise de préférence comme substance odorante, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

Selon un mode préféré de réalisation de l'invention, on utilise un mélange de différentes substances odorantes qui engendrent en commun une note parfumante plaisante pour l'utilisateur.

La phase grasse liquide de la composition est de préférence présente en une quantité d'au moins 20 % du poids total de la composition, allant par exemple de 20 à 88,5 % en poids, de préférence de 30 à 85 % en poids et mieux de 40 à 80 % en poids par rapport au poids total de la composition. Les huiles peuvent être polaires ou non polaires, de différentes natures chimiques, seules ou en mélange. Elles sont choisies de telle sorte qu'elles ne nuisent pas à la transparence de la composition.

Selon un mode préféré de réalisation de l'invention, la composition comprend au moins une huile polaire et au moins une huile non polaire.

En particulier, on peut citer comme huiles polaires :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearineries Dubois ou ceux vendus sous les dénominations Miglyol 810, 812 et 818 par la société Dynamit Nobel ;
- les esters et les éthers de synthèse comme le myristate d'isopropyle, des octanoates, décanoates ou ricinoléates d'alcools ou de polyalcools, notamment les esters d'acide gras en C₈-C₂₄ et de polyols comme le glycérol, le sorbitan, le glucose ou le méthylglucose, tels que les stéarates, isostéarates, hydroxystéarates et oléates de sorbitan, de glycéryle, de glucose ou de méthylglucose ; les huiles de synthèse de formule R₅COOR₆ dans laquelle R₅ représente le reste d'un acide gras supérieur linéaire ou ramifié comportant de 1 à 40 et de préférence de 7 à 19 atomes de carbone et R₆ représente une chaîne hydrocarbonée ramifiée contenant de 1 à 40 et de préférence de 3 à 20 atomes de carbone, à condition que le nombre d'atomes de carbone de R₅ + R₆ soit au moins de 10, comme par exemple l'huile de Purcellin (octanoate de cétostéaryle), l'isononanoate d'isononyle, le benzoate d'alcool en C₁₂ à C₁₅ ;
- les alcools gras saturés en C₁₂ à C₂₆ comme l'octyldodécanol, l'alcool isocétylique, l'alcool béhénylique ;
- les acides gras ;
- leurs mélanges.

Par ailleurs, les huiles non polaires peuvent être choisies en particulier parmi les huiles siliconées telles que les polydiméthylsiloxanes volatils ou non, linéaires ou cycliques, liquides à température ambiante ; les hydrocarbures ou fluorocarbures linéaires ou ramifiés d'origine synthétique ou minérale comme les huiles de paraffine volatiles (telles que les isoparaffines, l'isododécane) ou non volatiles et ses dérivés, la vaseline, les polydécènes, le polyisobutène hydrogéné tel que l'huile de parléam, le squalane.

Selon un mode préféré de réalisation de l'invention, la composition comprend au moins une huile choisie parmi les esters d'acide gras en C₈-C₂₄ et de polyols, et les alcools gras saturés en C₁₂ à C₂₆, et notamment de l'octyldodécanol.

La phase grasse peut aussi contenir d'autres corps gras comme par exemple les cires, dans la mesure où celles-ci sont compatibles avec les autres composants de la composition, et où elles n'altèrent pas les propriétés de la composition et notamment sa transparence.

La composition de l'invention peut comprendre, en outre, tout additif usuellement utilisé dans le domaine concerné, choisi notamment parmi les antioxydants, les conservateurs, les neutralisants, les actifs cosmétiques ou dermatologiques comme par exemple les émollients, les hydratants, les vitamines, les acides gras essentiels, les filtres solaires, et leurs mélanges. Quand ils sont présents dans la composition de l'invention, ces additifs peuvent être présents en une quantité allant de 0,001 à 20 % et mieux de 0,01 à 10% en poids par rapport au poids total de la composition. Avantageusement, la composition contient au moins un actif cosmétique ou dermatologique.

Bien entendu l'homme du métier veillera à choisir les éventuels additifs complémentaires et/ou leur quantité de telle manière que les propriétés avantageuses de la composition selon l'invention ne soient pas ou substantiellement pas altérées par l'adjonction envisagée.

L'invention s'applique non seulement aux produits parfumants mais aussi aux produits de soin, de traitement et/ou de maquillage de la peau, y compris du cuir chevelu, et des lèvres, contenant une substance odorante. La composition selon l'invention peut ainsi constituer une composition de parfumage, de soin, de traitement et/ou de maquillage des matières kératiniques, et notamment se présenter sous forme de stick transparent. Elle peut constituer par exemple des produits en stick de protection solaire de la peau du visage ; des produits démaquillants ; des produits de maquillage de la peau, aussi bien du visage que du corps humain, et notamment sous forme de produits de maquillage colorés comme les fonds de teints coulés en stick ou en coupelle, les blushs et fards à joues, les rouges à lèvres, les produits anti-cerne et les produits de tatouage éphémère ; des produits de maquillage des yeux, des sourcils et/ou des cheveux comme les eye-liners sous forme de crayon et les mascaras pains ; des produits d'hygiène corporelle. Elle est tout particulièrement appropriée comme produit parfumant.

L'invention a encore pour objet un procédé cosmétique de parfumage des matières kératiniques des êtres humains et notamment de la peau, des lèvres et des phanères, comprenant l'application sur les matières kératiniques de la composition telle que définie ci-dessus.

Selon un mode particulier de réalisation de l'invention, la composition peut contenir une matière colorante qui peut être choisie parmi les colorants lipophiles, les colorants hydrophiles habituellement utilisés dans les compositions cosmétiques ou dermatologiques, et leurs mélanges. Cette matière colorante peut être présente à raison de 0,001 à 5 % du poids total de la composition, de préférence de 0,01 à 2 % du poids total de la composition.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine.

La composition selon l'invention peut être fabriquée par les procédés connus, généralement utilisés dans le domaine cosmétique ou dermatologique. Elle peut être fabriquée par exemple par le procédé qui consiste à chauffer le polymère jusqu'à au moins sa température de ramollissement, à y ajouter les autres composés, les matières colorantes et les additifs, puis à mélanger le tout jusqu'à l'obtention d'une solution claire, transparente. Le mélange homogène obtenu peut alors être coulé dans un moule approprié comme un moule de rouge à lèvres ou directement dans les articles de conditionnement (boîtier, pot ou coupelle notamment).

L'invention est illustrée plus en détail dans les exemples suivants. Les pourcentages sont donnés en poids sauf mention contraire.

### Exemple 1 : Composition parfumante

- Uniclear 100 25 %
- Extrait de parfum 4 %
- Huile de parléam 61 %
- Octyldodécanol 10 %

Mode opératoire : on mélange les constituants sauf l'extrait de parfum, à chaud (environ 80°C) et on ajoute ensuite l'extrait de parfum. On agite, puis on coule et on laisse refroidir jusqu'à la température ambiante (20 à 25°C).

On obtient un stick parfaitement transparent et ayant une dureté dont la force est de 73 g. Ce stick est très parfumant, le parfum persistant durablement après application sur la peau.

Mesure de turbidité : la mesure est effectuée avec le turbidimètre portable 2100P de HACH COMPAGNY, le tube de mesure étant du type 2100P : AR397A, cat.24347-06 pk/6.

Pour effectuer la mesure, on coule la composition à mesurer dans le tube de mesure que l'on place dans le turbidimètre. La valeur de turbidité de cette composition est de 12,8 NTU.

### Test de rémanence :

Test comparatif n° 1 : On a mesuré la rémanence du stick de l'exemple 1 selon l'invention par rapport à une composition fluide contenant la même quantité d'extrait de parfum. La composition fluide est une émulsion huile-dans-eau parfumante utilisée couramment et comprenant 17 % en poids d'huiles, 7 % en poids de tensioactifs et co-tensioactifs, 4 % en poids d'extrait de parfum , 7 % en poids de glycérine, le complément à 100 % en poids étant de l'eau. .

Pour mesurer la rémanence des compositions étudiées, on met environ 25 mg de la composition dans un vial (petit flacon vissé et serti) de 4 ml, ayant une surface de contact de 40 mm², en équilibre d'évaporation dynamique sous un flux de 45 ml d'air par minute et à une température de 40°C. A 6 moments précis allant de 5 minutes à 6h10 après l'application de la composition, on quantifie la quantité de parfum libérée par le résiduel parfum de la composition étudiée.

Sur le tableau (1) suivant, les valeurs exprimées en unités d'intégration (UI.10⁻⁵) représentent la somme des aires des pics du parfum à chaque moment de la quantification.

**Tableau (1)**

| Composition | Mesure à 5mn | Mesure à 45 mn | Mesure à 1h33 | Mesure à 2h15 | Mesure à 4h12 | Mesure à 6h10 |
|---|---|---|---|---|---|---|
| Stick de l'exemple 1 | 13,15 | 5,64 | 3,78 | 3,12 | 1,73 | 0,87 |
| Fluide | 16,32 | 3,10 | 1,61 | 1,20 | 0,49 | 0,44 |

Ce tableau met en évidence qu'après 6 heures d'évaporation dynamique contrôlée, le stick anhydre selon l'invention libère deux fois plus de parfum que la composition fluide de l'art antérieur et que la quantité de parfum libérée par le stick après 4h12 est équivalente à celle libérée par la composition fluide après 1h33. Ainsi, l'effet de rémanence des sticks selon l'invention est environ trois fois plus important que celui de la composition fluide qui lui est comparée.

Test comparatif n° 2 : Selon le même test que ci-dessus, on a mesuré la rémanence du stick de l'exemple 1 selon l'invention dans lequel l'extrait de parfum était le limonène ou le citronellol, par rapport à une composition solide de l'art antérieur contenant la même quantité du même extrait de parfum. La composition solide de l'art antérieur comprenait 53,4 % en poids d'huile hydrocarbonée, 9 % en poids de cires, 4 % en poids d'extrait de parfum , 33,5 % en poids de charges, 0,1 % en poids de colorants et antioxydants.

Sur les tableaux (2) et (3) suivant, les valeurs exprimées en unités d'intégration (UI.10⁻³) représentent la somme des aires des pics du parfum à chaque moment de la quantification pour le limonène (tableau 2) et le citronellol (tableau 3).

**Tableau (2)**

| Composition au limonène | Mesure à 10 mn | Mesure à 1 h | Mesure à 1h 50 | Mesure à 3 h | Mesure à 4 h 30 | Mesure à 6 h |
|---|---|---|---|---|---|---|
| Composition solide de l'art antérieur | 18,8 | 6,3 | 3,1 | 1,1 | 0,28 | 0 |
| Stick de l'exemple 1 | 10,9 | 5,9 | 3,8 | 2 | 0,9 | 0,4 |

**Tableau (3)**

| Composition au citronellol | Mesure à 10 mn | Mesure à 1h | Mesure à 1 h 50 | Mesure à 3 h | Mesure à 4 h 30 | Mesure à 6 h |
|---|---|---|---|---|---|---|
| Composition solide de l'art antérieur | 18,9 | 9,7 | 6 | 2,8 | 1,09 | 0,53 |
| Stick de l'exemple 1 | 8,1 | 5,8 | 4,6 | 3,3 | 2,2 | 1,6 |

Les tableaux (2) et (3) montrent que le stick selon l'invention permet une libération plus progressive et plus persistante du parfum, et donc une meilleure rémanence dans le temps.

### Exemple 2 : Baume pour les lèvres parfumé à la violette

- Uniclear 100 25 %
- Parfum « Violine » 4 %
- Huile de parléam 60,998 %
- Octyldodécanol 10 %
- Colorant D&C violet n°2 0,002 %

Le mode opératoire est le même que dans l'exemple 1.

On obtient un baume en stick pour les lèvres, parfaitement transparent, coloré en violet, et parfumant agréablement les lèvres. Ce stick a une dureté dont la force est de 47 g.

La mesure de turbidité est réalisée de la même manière que dans l'exemple 1. On obtient une valeur de turbidité de 12,8 NTU.

### Exemple 3 : stick parfumant

- Versamid 940 25 %
- Parfum 2 %
- Octyldodécanol 73 %

Le mode opératoire est le même que dans l'exemple 1.

On obtient un stick parfaitement transparent, ayant une dureté dont la force est de 52,5 g.

La mesure de turbidité est réalisée de la même manière que dans l'exemple 1. On obtient une valeur de turbidité de 18,6 NTU.

### Exemple 4 : produit coulé parfumant

- Casamid 872 25 %
- Parfum 2 %
- Octyldodécanol 73 %

Le mode opératoire est le même que dans l'exemple 1.

On obtient un produit coulé souple transparent, présenté en coupelles et ayant une dureté dont la force est de 5 g.

La mesure de turbidité est réalisée de la même manière que dans l'exemple 1. On obtient une valeur de turbidité de 334 NTU.

### Exemple 5 : produit coulé parfumant

- Uni-Rez 100 20 %
- Parfum 2 %
- Octyldodécanol 78 %

Le mode opératoire est le même que dans l'exemple 1.

On obtient un produit souple transparent, présenté en coupelles et ayant une dureté dont la force est de 6 g.

La mesure de turbidité est réalisée de la même manière que dans l'exemple 1. On obtient une valeur de turbidité de 7,98 NTU.

## Revendications

1. Composition cosmétique solide anhydre transparente contenant dans un milieu physiologiquement acceptable, au moins 2 % en poids par rapport au poids total de la composition, d'une ou plusieurs substances odorantes, et une phase grasse liquide contenant au moins un polymère de masse moléculaire moyenne en poids allant de 1.000 à 30.000, comportant a) un squelette polymérique ayant des motifs de répétition hydrocarbonés pourvus d'au moins un hétéroatome, et b) au moins une chaîne grasse pendante et/ou terminale éventuellement fonctionnalisée, ayant de 12 à 120 atomes de carbone et étant liée à ces motifs hydrocarbonés.

2. Composition selon la revendication 1, **caractérisée en ce que** la phase grasse liquide comprend au moins une huile polaire et une huile non polaire.

3. Composition selon la revendication 1 ou 2, **caractérisée en ce qu'**elle comprend au moins une huile choisie parmi les esters d'acide gras en C₈-C₂₄ et de polyols, et les alcools gras saturés en C₁₂ à C₂₆.

4. Composition selon l'une quelconque des revendications précédentes, **caractérisée en ce qu'**elle comprend de l'octyldodécanol.

5. Composition selon l'une quelconque des revendications précédentes, **caractérisée en ce que** les motifs à hétéroatome sont des amides.

6. Composition selon l'une quelconque des revendications précédentes, **caractérisée en ce que** les chaînes grasses représentent de 40 à 98 % du nombre total des motifs à hétéroatome et des chaînes grasses.

7. Composition selon l'une des revendications précédentes, **caractérisée en ce que** les chaînes grasses représentent de 50 à 95 % du nombre total des motifs à hétéroatome et des chaînes grasses.

8. Composition selon l'une des revendications précédentes, **caractérisée en ce que** la ou les chaînes grasses pendantes sont liées directement à l'un au moins desdits hétéroatomes.

9. Composition cosmétique solide anhydre transparente contenant dans un milieu physiologiquement acceptable, au moins 2 % en poids d'au moins une substance odorante, et une phase grasse liquide contenant au moins un polyamide de masse moléculaire moyenne en poids allant de 1.000 à 30.000, comportant a) un squelette polymérique ayant des motifs répétitifs amide, et b) éventuellement au moins une chaîne grasse pendante et/ou terminale éventuellement fonctionnalisée, ayant de 12 à 120 atomes de carbone et étant liée à ces motifs amide.

10. Composition selon la revendication 9, **caractérisée en ce que** le polyamide comporte une ou plusieurs chaînes grasses pendantes liées directement à l'un au moins des atomes d'azote des motifs amide.

11. Composition selon la revendication 9 ou 10, **caractérisée en ce que** le polyamide comporte une ou plusieurs chaînes grasses terminales liées au squelette par des groupes ester.

12. Composition selon la revendication 10 ou 11, **caractérisée en ce que** les chaînes grasses représentent de 40 à 98 % du nombre total des motifs amide et des chaînes grasses.

13. Composition selon la revendication précédente, **caractérisée en ce que** les chaînes grasses représentent de 50 à 95 % du nombre total des motifs amide et des chaînes grasses.

14. Composition selon l'une quelconque des revendications précédentes, **caractérisée en ce que** les chaînes grasses ont de 12 à 68 atomes de carbone.

15. Composition selon l'une des revendications précédentes, **caractérisée en ce que** le polymère a une masse moléculaire moyenne en poids de 2.000 à 20.000 et mieux de 2.000 à 10.000.

16. Composition selon l'une des revendications précédentes, **caractérisée en ce que** le polymère est choisi parmi les polymères de formule (I) suivante et leurs mélanges : 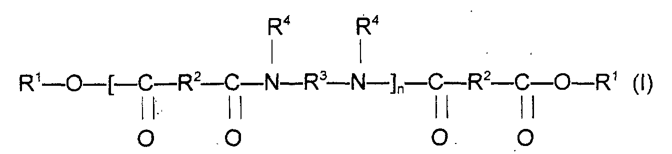 dans laquelle n désigne un nombre entier de motifs amide, tel que le nombre de groupes ester représente de 10 à 50 % du nombre total des groupes ester et amide ; R¹ est à chaque occurrence indépendamment un groupe alkyle ou alkényle ayant au moins 4 atomes de carbone ; R² représente à chaque occurrence indépendamment un groupe hydrocarboné en C₄ à C₄₂ à condition que au moins 50 % des groupes R² représentent un groupe hydrocarboné en C₃₀ à C₄₂; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.

17. Composition selon la revendication précédente, **caractérisée en ce que** R¹ est un groupe alkyle en C₁₂ à C₂₂.

18. Composition selon l'une des revendications 16 ou 17, **caractérisée en ce que** R² sont des groupes ayant de 30 à 42 atomes de carbone.

19. Composition selon l'une quelconque des revendications précédentes, **caractérisée en ce que** le polymère est choisi parmi les copolymères d'un diacide en C₃₆ condensé sur l'éthylène diamine, estérifiés par l'alcool cétylstéarylique ou l'alcool stéarylique ; les résines polyamides résultant de la condensation d'un acide dicarboxylique aliphatique et d'une diamine, les groupes carbonyle et amine de motifs unitaires adjacents étant condensés par une liaison amide; les polyamides de dimères d'acides gras et de diamines aliphatiques ; les polyamides à base d'acides gras dimères, et leurs mélanges.

20. Composition selon l'une quelconque des revendications précédentes, **caractérisée en ce que** le polymère représente de 0,5 à 50 % du poids total de la composition et de préférence de 5 à 40 % du poids total de la composition.

21. Composition selon l'une quelconque des revendications précédentes, **caractérisée en ce que** la substance odorante est choisie parmi les parfums et les arômes d'origine naturelle ou synthétique, et leurs mélanges.

22. Composition selon l'une quelconque des revendications 15 % en poids et de préférence de 3 à 12 % par rapport au poids total de la composition.

23. Composition selon l'une quelconque des revendications précédentes, **caractérisée en ce que** la phase grasse liquide est présente en une quantité d'au moins 20 % du poids total de la composition.

24. Composition selon l'une quelconque des revendications précédentes, **caractérisée en ce que** la phase grasse liquide représente une quantité de 20 à 88,5 % en poids par rapport au poids total de la composition.

25. Composition selon l'une quelconque des revendications précédentes, **caractérisée en ce qu'**elle constitue une composition de parfumage, de soin et/ou de traitement et/ou de maquillage des matières kératiniques.

26. Composition selon l'une quelconque des revendications précédentes, **caractérisée en ce qu'**elle présente une dureté dont la force va de 5 à 600 grammes.

27. Composition selon l'une quelconque des revendications précédentes, **caractérisée en ce qu'**elle se présente sous forme d'un stick transparent ou d'un produit coulé.

28. Composition selon l'une quelconque des revendications précédentes, **caractérisée en ce qu'**elle constitue un produit parfumant.

29. Procédé cosmétique de parfumage des matières kératiniques des êtres humains, comprenant l'application sur les matières kératiniques d'une composition selon à l'une quelconque des revendications 1 à 28.

30. Utilisation d'une quantité suffisante d'au moins un polymère de masse moléculaire moyenne en poids allant de 1.000 à 30.000, comportant a) un squelette polymérique ayant des motifs de répétition hydrocarbonés pourvus d'au moins un hétéroatome, et b) des chaînes grasses pendantes et/ou terminales éventuellement fonctionnalisées, ayant de 12 à 120 atomes de carbone et étant liées à ces motifs hydrocarbonés, dans une composition contenant dans un milieu physiologiquement acceptable une substance odorante, pour augmenter la rémanence de ladite substance odorante.

31. Utilisation d'une quantité suffisante d'au moins un polyamide de masse moléculaire moyenne en poids allant de 1.000 à 30.000, comportant a) un squelette polymérique ayant des motifs répétitifs amide, et b) éventuellement des chaînes grasses pendantes et/ou terminales éventuellement fonctionnalisées, ayant de 12 à 120 atomes de carbone et étant liées à ces motifs amide, dans une composition contenant dans un milieu physiologiquement acceptable une substance odorante, pour augmenter la rémanence de ladite substance odorante.

32. Utilisation selon la revendication 30 ou 31, **caractérisée en ce que** le polymère est choisi parmi les polymères de formule (I) suivante et leurs mélanges : 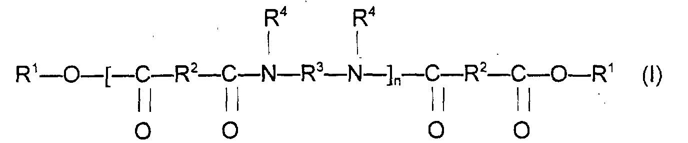 dans laquelle n désigne un nombre entier de motifs amide, tel que le nombre de groupes ester représente de 10 à 50 % du nombre total des groupes ester et amide ; R¹ est à chaque occurrence indépendamment un groupe alkyle ou alcényle ayant au moins 4 atomes de carbone ; R² représente à chaque occurrence indépendamment un groupe hydrocarboné en C₄ à C₄₂ à condition que 50 % des groupes R² représentent un groupe hydrocarboné en C₃₀ à C₄₂ ; R³ représente à chaque occurrence indépendamment un groupe organique pourvu d'au moins 2 atomes de carbone, d'atomes d'hydrogène et optionnellement d'un ou plusieurs atomes d'oxygène ou d'azote ; et R⁴ représente à chaque occurrence indépendamment un atome d'hydrogène, un groupe alkyle en C₁ à C₁₀ ou une liaison directe à R³ ou à un autre R⁴ de sorte que l'atome d'azote auquel sont liés à la fois R³ et R⁴ fasse partie d'une structure hétérocyclique définie par R⁴-N-R³, avec au moins 50 % des R⁴ représentant un atome d'hydrogène.
